# EUROPEAN PATENT SPECIFICATION

(11) **EP 1 181 983 B1**
(45) Date of publication and mention of the grant of the patent: **07.01.2009**
(21) Application number: 01307151.9
(22) Date of filing: 22.08.2001
(51) Int. Cl.: B05B 7/06, A61K 9/28

(54) **Spray gun for enteric coating and process for producing enteric coated preparation**
Sprühpistole für darmsaftlösliche Beschichtungsmaterialien und Verfahren zur Erzeugung von mit diesen Materialien beschichteten Präparaten
Pistolet de pulvérisation pour produits de revêtement entérosolubles et procédé de production de préparations revêtues de tels produits

(30) Priority: 25.08.2000 JP 2000254836
(43) Date of publication of application: 27.02.2002
(73) Proprietor: Shin-Etsu Chemical Co., Ltd., Chiyoda-ku, Tokyo (JP)
(72) Inventor: Nishiyama, Yuichi, c/o Specialty Chemicals Research Center, Kubiki-mura, Naka Kubiki-gun, Niigata-ken (JP); Hayakawa, Kazuhisa, c/o Specialty Chemicals Research Center, Kubiki-mura, Naka Kubiki-gun, Niigata-ken (JP)
(74) Representative: Goddard, David John

(56) References cited:
- DE-B- 1 079 998
- DATABASE WPI Derwent Publications Ltd., London, GB; AN 1984-233931 XP002219898 & JP 59 139266 A (SHINETSU CHEM IND CO LTD )
- PATENT ABSTRACTS OF JAPAN vol. 007, no. 277 (C-199), 9 December 1983 (1983-12-09) & JP 58 157726 A (SHINETSU KAGAKU KOGYO KK), 19 September 1983 (1983-09-19)

## Description

### BACKGROUND OF THE INVENTION

### 1. Field of the Invention

The present invention relates to an improved process for producing an enteric coated preparation.

### 2. Description of the Related Art

As a process for producing enteric coated preparations, conventionally and widely known is a process for applying a coating solution, which has been obtained by dissolving an enteric coating material in an organic solvent, to tablets, granules or capsules. Since this process requires use of a large amount of an organic solvent, however, it is accompanied with the problems such as a danger of fire or explosion, a threat to safety and health for workers, and environmental pollution due to dispersion of the organic solvent in the air. In addition, it is disadvantageous in cost.

Accordingly, there is a demand for the development of art for producing an enteric coated preparation without using an organic solvent. From this viewpoint, Japanese Patent Provisional Publication (JP-A) No. 55-98120/1980 (U.S. Patent No. 4,287,221) discloses a process wherein a coating solution is obtained by dispersing hydroxypropyl methyl cellulose phthalate powders having an average particle size of 100 µm or less in water of 25°C or less containing triacetin, and sprayed from a spray nozzle to a preparation, while the resulting sprayed preparation is simultaneously dried. Japanese Patent Publication (JP-B) No. 3-80767/1991, discloses a process wherein triethyl citrate as a plasticizer is used.

The above-described processes permit coating without using an organic solvent. However, they have a problem of clogging the spray gun. Since a pan in which preparations are rolled is under a heated condition for drying, a solution of a coating composition is heated in a pipe and a nozzle in the pan, the pipe being for leading the solution to a spray nozzle placed in the pan. Consequently, a plasticizer and an enteric polymer happen to form agglomerates owing to their interaction so that the spray gun is clogged.

With a view to overcoming the problem, Japanese Patent Publication (JP-B) No. 5-9407/1993 discloses a process wherein an enteric polymer dispersion is sprayed from one spray gun, while simultaneously a plasticizer solution is sprayed from another spray gun. However, this process is accompanied with the problems that it requires two spray guns which increases an equipment installation cost, and that the yield of the polymer sprayed is inferior because it takes time to obtain a uniform mixture with the plasticizer.

DE 1079998B discloses a two-stream spray gun having an inside nozzle 3, an outside nozzle 4, and a mixing room 15 for coating a substance with lacquer.

JP 59139266A describes the spraying of an aqueous dispersion of powder of an enteric coating agent from one spray gun, and an aqueous solution or dispersion of a plasticizer from another, different spray gun.

JP 58157726A describes dispersing a fine powder of a coating base in an aqueous medium containing a plasticizer and a water-soluble or dispersible macro molecular substance, in this instance only a single dispersion is prepared and sprayed.

### SUMMARY OF THE INVENTION

An object of the present invention is to provide an enteric coating technique capable of applying enteric coating to preparations even without using an organic solvent and without causing a spray gun to clog, while keeping an equipment cost low.

With a view toward overcoming the above-described problems, the present inventors have carried out an extensive investigation. As a result, provided is a method of producing an enteric coated preparation according to claim 1 and dependent claims.

In the method, an enteric polymer dispersion and/or solution and a plasticizer liquid are sprayed simultaneously but separately from separate nozzles of a spray gun so that they can be mixed during spraying. Consequently, preparations having a gastro-resistant but enteric coatings are obtained without clogging a spraying gun and without using an organic solvent, while keeping an equipment coat low. Thus, the present inventors have completed the invention.

According to the present invention, coating can be effected without clogging a spray gun and without using an organic solvent, while keeping an equipment cost low.

### BRIEF DESCRIPTION OF THE DRAWINGS

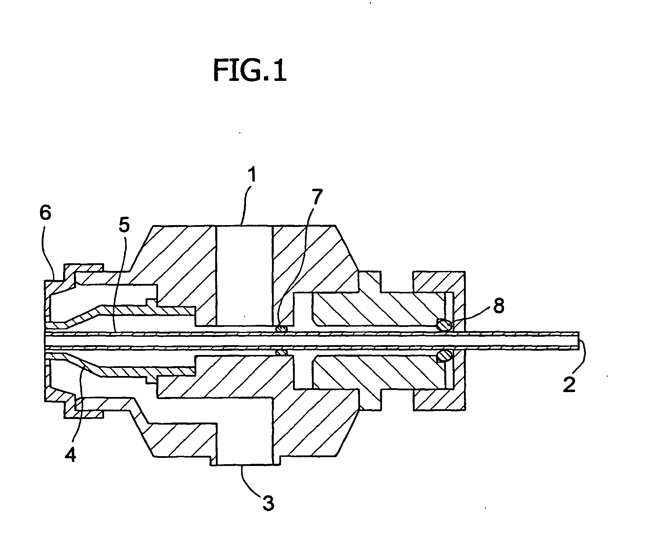

Fig. 1 is a cross-sectional view illustrating one example of a spray gun according to the present invention.

### DETAILED DESCRIPTION OF THE PREFERRED INVENTION

The present invention will hereinafter be described more specifically. However, it should be construed that the present invention is not limited to the following embodiments.

One example of a spray gun according to the present invention is illustrated in Fig. 1

The spray gun has a construction such that an enteric polymer dispersion and/or enteric polymer solution and a plasticizer solution, which can be selected suitably, can be introduced by a pump from Inlet 1 for Liquid A and Inlet 2 for Liquid B. Inlet 1 for Liquid A and Inlet 2 for Liquid B are connected with nozzle openings of Nozzle-A 4 and Nozzle-B 5, respectively, from which Liquids A and B are discharged by spraying. Air from Air inlet 3 is discharged from the nozzle opening of Air nozzle (cap) 6. In FIG. 1, the nozzle opening of Nozzle-A 4, the nozzle opening of Nozzle-B 5 and the nozzle opening of Air nozzle form substantially concentric circles, being arranged from the inside toward the outside. O-ring 7 and packing 8 are also shown in FIG.1. Liquid A may be an enteric polymer dispersion and/or enteric polymer solution, and Liquid B may be a plasticizer solution. Alternatively, Liquid A may be the plasticizer solution and Liquid B may be the enteric polymer dispersion and/or enteric polymer solution.

No particular limitation is imposed on the pump for introducing the enteric polymer dispersion and/or solution and the plasticizer solution. A conventionally employed pump can be used, while the preferred pump is, for example, a gear pump or a tube pump.

Although no particular limitation is imposed on the quality of the material of the spray gun used in the present invention insofar as it has water resistance and is not dissolved or melted in a plasticizer at room temperature to about 100°C, a heat resistant and anticorrosive material such as stainless steel or silicone is preferred. A water system is preferred in consideration of fire or explosion risk elimination, safety and health of workers, and environmental protection. In the case where an organic solvent is used, organic solvent resistance is required.

Although there is no particular limitation imposed on the shape or diameter of the nozzle insofar as spraying can be conducted, a diameter or wall-to-wall distance of a pipe of about 0.1 to 5 mm which can facilitate spraying is preferred.

Although no particular limitation is imposed on the liquid feed rate of the spray gun, the liquid feed rate of several grams per minute to several hundred grams per minute is preferred, because it is a measure for smooth coating. The spray gun may have a construction such that air or gas can be fed to the nozzle for spraying. Although no particular limitation is imposed on the feed rate of this air or gas insofar as spraying can be conducted, the feed rate of several tens to several hundreds liters per minute is preferred. Although no particular limitation is imposed on the kind of the gas other than air, inert gases free from interaction with a medicament such as nitrogen and helium are preferred.

The conventional enteric polymer may be used in the present invention. Examples thereof include hydroxypropyl methyl cellulose phthalate (HPMCP) and cellulose acetate phthalate (CAP) each specified in Japanese Pharmacopoeia, and hydroxypropyl methyl cellulose acetate succinate (HPMCAS), carboxymethyl ethyl ether (CMEC) and methyl acrylate-ethyl methacrylate copolymer (another name: Eudragit) each specified in Japanese Pharmaceutical Excipients Standards. Although no particular limitation is imposed on the particle size of such an enteric polymer used in the form of a dispersion insofar as it does not clog the spray gun, the average particle size is usually 100 µm or less, preferably 50 µm or less. The enteric polymer may also be used after being dissolved in weakly alkali water such as aqueous ammonia.

In the present invention, the enteric polymer dispersion may be prepared by dispersing the enteric polymer in a predetermined amount of water while stirring. Although no particular limitation is imposed on the concentration of the dispersion, the concentration of 5 to 30% by weight is preferred. This concentration range may be also preferrably applicable to the case where the enteric polymer is used not in a dispersion but in a solution having the enteric polymer dissoved in weakly alkali water such as aqueous ammonia.

In the present invention, the plasticizer liquid may be any one of only a plasticizer, a dispersion of the plasticizer, a solution of the plasticizer and a mixture of the plasticizer dispersion and plasticizer solution.

Examples of the plasticizer used in the present invention include triethyl citrate, tributyl citrate, tributyl acetylcitrate, triethyl acetylcitrate, propylene glycol, dipropylene glycol, triacetin, diacetin, monoacetin, benzyl alcohol, diethyl phthalate, dibutyl phthalate, glycerin phthalate, polyethylene glycol, polyoxyethylene alkyl ethers, polyethylene glycol fatty acid esters and propylene glycol fatty acid esters.

These plasticizers may be used either singly or in combination. They may be dispersed or dissolved in water prior to coating.

These plasticizers are added to improve the plasticity of the enteric polymer, thereby improving uniformity of the coating film. Although no particular limitation is imposed on the amount of the plasticizer insofar as it is enough to attain the improvement, a range of 5 to 60 wt% based on the amount of the enteric polymer is preferred.

The coating amount differs depending on the kind of the solid dosage form (solid preparation), but 3 to 50 wt%, in terms of solid coating portion, based on the weight of the solid dosage form is preferred. Prior to application to the solid dosage form, coating material, for example, a gastro-soluble coating material such as hydroxypropyl methyl cellulose may be applied to the solid dosage form. Consequently, enteric coating of the solid dosage form which tends to be broken by an impact can be also accomplished with even a small amount of coating material, while satisfying gastro-resistance.

Although no particular limitation is imposed on the form of the preparation to be coated, solid preparations such as tablets, granules and capsules are preferred for uniform coating.

Although a pan coating apparatus, a coating apparatus equipped with air drying mechanism or a fluid coating apparatus can be employed as a coating apparatus, it is necessary to use a spray gun of the present invention which can spray two liquids from separate nozzles.

The present invention will be described below in further details by Example. However, It should be construed that the present invention is not limited to the example.

### Example 1

Coating was applied to tablets, each containing lactose, corn starch and Japanese Pharmacopoeia L-HPC ("LH-1" of Shin-Etsu Chemical Co., Ltd.), having a diameter of 8 mm and weight of 190 mg, under the below-described conditions. Thus, enteric coated tablets were obtained. As an enteric coating material, HPMCAS fine powders ("AS-MF" of Shin-Etsu Chemical Co., Ltd., having average particle size of 5 µm) were employed, while triethyl citrate was employed as a plasticizer.

The enteric polymer dispersion:

| | |
|---|---|
| AS-MF | 15 parts by weight |
| Talc | 4.5 parts by weight |
| Water | 80.5 parts by weight |
| Plasticizer: | triethyl citrate 100 parts by weight |

Coating was conducted under the following operation conditions using the above two coating solutions.

Coating apparatus: "HICOATER HCT-48N" of Freund Inc.
Spray gun: having a similar structure to that illustrated in FIG. 1. Nozzle A having an inner diameter of 2.5 mm, and Nozzle B having an outer diameter of 2.0 mm and inner diameter of 1.0 mm were employed.
   Nozzles A and B, the enteric polymer dispersion and plasticizer were sprayed, respectively.
Transport pump
   Gear pump for enteric polymer dispersion
   Tube pump for plasticizer
Amount of tablets charged: 5 kg
Temperature of enteric polymer dispersion and plasticizer as coating liquids : 27°C
Drying air temperature: 80°C
Feed rate of air to spray gun: 140 liters/min
Feed rate of enteric polymer dispersion: 50 g/min
Feed rate of plasticizer: 2.1 g/min
   Amounts of HPMCAS and triethyl citrate: corresponding
   to 100:28 (weight ratio)
Coating time: 80 minutes
Amount of HPMCAS: 600 g (12% based on tablets)

The enteric coated tablets thus obtained were found to have smooth and beautiful appearance. When disintegration test was made according to the Japanese Pharmacopoeia, they underwent no change at the test using a first liquid, but disintegrated completely in 9 to 12 minutes at the test using a second liquid. Thus, the obtained enteric coated tablets satisfied the requirement as an enteric preparation.

## Claims

1. A process for producing an enteric coated preparation wherein an enteric polymer dispersion and/or solution and a plasticizer liquid are simultaneously sprayed onto a preparation **characterised in that** the enteric polymer dispersion and/or solution is sprayed from a first nozzle of a spray gun and the plasticizer liquid from a second nozzle of the spray gun, thereby coating the preparation.

2. A process for producing an enteric coated preparation in accordance with claim 1, wherein said enteric polymer is selected from the group comprising: hydroxypropyl methyl cellulose phthalate, hydroxypropyl methyl cellulose acetate succinate, cellulose acetate phthalate, carboxymethyl ethyl cellulose, and a water dispersible copolymer obtained by emulsion polymerization of ethyl acrylate and methacrylic acid.

3. A process according to claim 1 or claim 2 wherein the plasticizer in said plasticizer liquid is selected from at least one of the group comprising: triethyl citrate, tributyl citrate, tributyl acetylcitrate, triethyl acetylcitrate, propylene glycol, dipropylene glycol, triacetin, diacetin, monoacetin, benzyl alcohol, diethyl phthalate, dibutyl phthalate, glycerin phthalate, polyethylene glycol, polyoxyethylene alkyl ethers, polyethylene glycol fatty acid esters and propylene glycol fatty acid esters.

4. A process according to any one of preceding claims 1 to 3 wherein a concentration of said enteric polymer in said dispersion or solution lies in the range from 5% to 30% by weight.

5. A processing according to any one preceding claim from 1 to 4 wherein a concentration of said plasticizer liquid lies in a range from 5% to 60% by weight based on the amount of said enteric polymer.

## Patentansprüche

1. Verfahren zum Herstellen eines enterisch beschichteten Präparats, bei dem eine enterische Polymerdispersion und/oder -lösung und eine Weichmacherflüssigkeit gleichzeitig auf ein Präparat aufgesprüht werden, **dadurch gekennzeichnet, daß** die enterische Polymerdispersion und/oder -lösung aus einer ersten Düse einer Sprühpistole und die Weichmacherflüssigkeit aus einer zweiten Düse der Sprühpistole gesprüht werden und **dadurch** das Präparat beschichtet wird.

2. Verfahren zum Herstellen eines enterisch beschichteten Präparats nach Anspruch 1, bei dem das enterische Polymer ausgewählt ist aus der Gruppe, umfassend: Hydroxypropylmethylcellulosephthalat, Hydroxypropylmethylcelluloseacetatsuccinat, Celluloseacetatphthalat, Carboxymethylethylcellulose, und ein wasser-dispergierbares Copolymer, erhalten durch Emulsionspolymerisation von Ethylacrylat und Methacrylsäure.

3. Verfahren nach Anspruch 1 oder Anspruch 2, bei dem der Weichmacher in der Weichmacherflüssigkeit ausgewählt ist unter mindestens einer Verbindung aus der Gruppe, umfassend: Triethylcitrat, Tributylcitrat, Tributylacetylcitrat, Triethylacetylcitrat, Propylenglykol, Dipropylenglykol, Triacetin, Diacetin, Monoacetin, Benzylalkohol, Diethylphthalat, Dibutylphthalat, Glycerinphthalat, Polyethylenglykol, Polyoxyethylenalkylether, Polyethylenglykol-Fettsäureester und Propylenglykol-Fettsäureester.

4. Verfahren nach einem der vorhergehenden Ansprüche 1 bis 3, bei dem eine Konzentration des enterischen Polymers in der Dispersion oder Lösung im Bereich von 5 bis 30 Gew.-% liegt.

5. Verfahren nach einem vorhergehenden Anspruch 1 bis 4, bei dem eine Konzentration der Weichmacherflüssigkeit, bezogen auf die Menge des enterischen Polymers, in einem Bereich von 5 bis 60 Gew.-% liegt.

## Revendications

1. Procédé pour la production d'une préparation pour revêtements entériques dans lequel une dispersion et/ou solution de polymères entériques et un liquide de plastification sont simultanément vaporisés sur une préparation, **caractérisé en ce que** la dispersion et/ou solution polymère entérique est vaporisée à partir d'une première buse d'un pistolet de vaporisation et le liquide de plastification à partir d'une seconde buse du pistolet de vaporisation, revêtant ainsi la préparation.

2. Procédé pour la production d'une préparation à revêtement entérique, selon la revendication 1, **caractérisé en ce que** ledit polymère entérique est choisi parmi les groupes comprenant : hydroxypropyl méthyle cellulose phtalate, hydroxyle méthyle cellulose acétate succinate, cellulose acétate succinate, cellulose acétate phtalate, carboxylméthyle éthyl cellulose et un co-polymère soluble dans l'eau obtenu par polymérisation émulsion d'éthyl acrylate et d'acide méthacrylique.

3. Procédé selon la revendication 1 ou 2, **caractérisé en ce que** le composant de plastification dans ledit liquide de plastification est choisie parmi au moins un élément du groupe comprenant : tryéthyle citrate, tributyle acétylcitrate, triéthyle acétylcitrate, propylène glycol, dipropylène glycol, triacétine, diacétine, monoacétine, benzyle alcool, diéthyle phtalate, dibutyle phtalate, glycérine phtalate, polyéthylène glycol, éther polyoxyéthylène alkyl, polyéthylène glycol acide gras esters et propylène glycol acide gras esters.

4. Procédé selon l'une des revendications 1 à 3, **caractérisé en ce qu'**une concentration dudit polymère entérique dans ladite dispersion ou solution se trouve dans la gamme comprise entre 5 et 30% en poids.

5. Procédé selon l'une des revendications 1 à 4, **caractérisé en ce qu'**une concentration dudit liquide de plastification se trouve dans la gamme entre 5% et 60% en poids, basée sur la quantité dudit polymère entérique.
